# EUROPEAN PATENT SPECIFICATION

(11) **EP 1 857 082 B1**
(45) Date of publication and mention of the grant of the patent: **23.12.2009**
(21) Application number: 07015811.8
(22) Date of filing: 02.06.2000
(51) Int. Cl.: A61F 2/84

(54) **Stent delivery system for branched vessel**
Stentverabreichungssystem für verzweigte Blutgefässe
Système de livraison d'extenseurs pour vaisseaux sanguins ramifiés

(30) Priority: 04.06.1999 US 325996; 06.12.1999 US 455299
(43) Date of publication of application: 21.11.2007
(62) Divisional of application: 04078241.9
(73) Proprietor: Advanced Stent Technologies, Inc., Pleasanton, CA 94566 (US)
(72) Inventor: Vardi, Gil M., Chesterfield MO 63017 (US); Davidson, Charles J., Winnetka IL 60093 (US); Williams, Eric, Fairfield CA 94533 (US)
(74) Representative: Vossius & Partner

(56) References cited:
- EP-A- 0 897 700
- EP-A- 0 904 745
- WO-A-96/34580

## Description

### TECHNICAL FIELD

The present invention relates to catheter systems for delivering stents.

### BACKGROUND OF THE INVENTION

A type of endoprosthesis device, commonly referred to as a stent, may be placed or implanted within a vein, artery or other tubular body organ for treating occlusions, stenoses, or aneurysms of a vessel by reinforcing the wall of the vessel or by expanding the vessel. Stents have been used to treat dissections in blood vessel walls caused by balloon angioplasty of the coronary arteries as well as peripheral arteries and to improve angioplasty results by preventing elastic recoil and remodeling of the vessel wall. Two randomized multicenter trials have recently shown a lower restenosis rate in stent treated coronary arteries compared with balloon angioplasty alone (Serruys, PW et al., New England Journal of Medicine 331: 489-495 (1994) and Fischman, DL et al. New England Journal of Medicine 331:496-501 (1994)). Stents have been successfully implanted in the urinary tract, the bile duct, the esophagus and the tracheo-bronchial tree to reinforce those body organs, as well as implanted into the neurovascular, peripheral vascular, coronary, cardiac, and renal systems, among others. The term "stent" as used in this Application is a device which is intraluminally implanted within bodily vessels to reinforce collapsing, dissected, partially occluded, weakened, diseased or abnormally dilated or small segments of a vessel wall.

One of the drawbacks of conventional stents is that they are generally produced in a straight tubular configuration. The use of such stents to treat diseased vessels at or near a bifurcation (branch point) of a vessel may create a risk of compromising the degree of patency of the main vessel and/or its branches, or the bifurcation point and also limits the ability to insert a branch stent into the side branch if the result of treatment of the main, or main, vessel is suboptimal. Suboptimal results may occur as a result of several mechanisms, such as displacing diseased tissue, plaque shifting, vessel spasm, dissection with or without intimal flaps, thrombosis, and embolism.

WO 96/34580 discloses an endoprosthesis for the treatment of blood vessel bifurcation stenosis that is characterized by the fact that it comprises three tubular sections and two connectors. The three tubular sections comprise a proximal section, a first distal section aligned at least approximately with the proximal section and a second distal section. The first distal section is intended for insertion into a first blood vessel, which branches off on the bifurcation, wherein the first distal section is linked to the proximal section by a first lateral connector. The second distal section is located at the side of the first distal section and is intended for insertion into a second vessel, which branches off from the bifurcation, wherein the two distal sections have their proximal ends linked by a second connector.

As described in PCT Patent Application Publication No. WO 99/00835 filed 01/14/98; systems have been developed for deploying a main stent in a main vessel at the intersection of a main vessel and a branch vessel with a branch stent extending into a branch vessel through a side opening in the main stent. Unfortunately, several difficulties exist when attempting to position such an arrangement of a main and branch stents at a vessel intersection.

For example, in various approaches, the insertion of separate guidewires into both the main and branch vessels is required before the positioning of a main stent in a main vessel with a branch stent projecting through a side opening in the main stent into a branch vessel. Specifically, main and branch stents are advanced over the separate guidewires which have been pre-guided one after another into the respective main and branch vessels, such that the main stent can be deployed within the main vessel and the branch stent can be deployed (passing through the side opening in the main stent into the branch vessel). Unfortunately, when attempting to guide two separate guidewires through the main vessel such that one enters the branch vessel, the two guidewires typically tend to wrap around one another and become entangled. Accordingly, time and effort is required to individually position each of the two guidewires one after another.

An additional disadvantage of positioning conventional stents is the difficulty in visualizing the stents during and after deployment, and in general, the fact that they arc not readily imaged by low-cost and easy methods, such as x-ray or ultrasound imaging.

### SUMMARY OF THE INVENTION

As will be explained, an advantage of the present system is that it may be used for deploying a main stent in a main vessel with a side hole in the main stent positioned at a branch vessel. Optionally, the system may further position a branch stent in the branch vessel, wherein the branch stent is deployed through an opening in the side of the main stent with the side opening being in registry with the ostium of the branch vessel. In various aspects of the present invention, another advantage of the present system is that it avoids having to separately position first and second guidewires within the respective main and branch vessels prior to deployment of main and branch stents thereover. Rather, in various aspects of the present invention, only a single guidewire needs to initially be placed within the main vessel, with the present system subsequently deploying both the main and branch stents thereover.

In addition, methods of positioning a main stent at a vessel bifurcation such that a side opening in the main stent is positioned in registry with the ostium of a branch vessel are described. A first guidewire can be positioned in the main vessel such that a distal end of the main guidewire extends past the bifurcation. Thereafter, the present system, (comprising either a main catheter with a slidably positionable side sheath or side catheter, or a main catheter with an attached, non-sliding, side sheath or side catheter), can be advanced to a position proximate the bifurcation, wherein the main catheter can be advanced over the main guidewire, and wherein the main stent can be positioned over the main catheter. The distal end of the flexible side sheath (or second catheter can be positioned to pass through the interior of the main stent, (which positioned over the distal end of the main catheter), and out of the side opening in the main stent.

Thereafter, a branch guidewire can be advanced through the flexible side sheath (or second catheter) into the branch vessel. To assist in guiding the second guidewire into the branch vessel, the flexible side sheath (or second catheter) may taper to a narrow distal end, which may also be curved slightly outwardly.

Subsequently, the system can be advanced with the main catheter advancing over the first guidewire as the flexible side sheath (or second catheter) concurrently advances over the second (ie: branch) guidewire. The side opening in the main stent can be positioned in alignment with the ostium of the branch vessel due solely to the presence of the second guidewire extending from an interior of the main stent out through the side opening in the main stent and into the branch vessel.

In certain examples (in which the flexible side sheath or second catheter is slidably moveable with respect to a main catheter), the second lumen of the dual lumen catheter (through which the flexible side sheath or second catheter passes) is formed from pebax and graphite, thereby reducing sliding friction when the flexible side sheath (or second catheter) is passed therethrough. Accordingly, the distal end of the side sheath, (which is received through the second lumen of the dual lumen catheter), can easily be slidably positioned to a desired location at the intersection of the main and branch vessels such that the dual lumen catheter can be positioned at a desired location to deploy the main stent within the main vessel.

In certain examples (in which the flexible side sheath or second catheter is not slidably moveable with respect to the main catheter, but is instead attached thereto), the distal end of the side sheath, (or second catheter), is positioned at a desired location at the intersection of the main and branch vessels by advancing the main catheter over the first guidewire in the main vessel such that the main catheter can be positioned at a desired location to deploy the main stent within the main vessel.

In both examples, after the distal end of the side sheath (or second catheter) is positioned at the vessel intersection, a second guidewire can then be advanced through the side sheath (or second catheter) to pass out of the distal end of the side sheath (or second catheter), passing through the side opening in the main stent and into the branch vessel, thereby aligning the side opening of the main stent in registry with the ostium of the branch vessel. Thereafter, the main stent may be deployed within the main vessel, such as by inflating a first balloon disposed over the first guidewire at a distal end of the main catheter. The delivery system of the present invention may be employed in a method of delivering main and branch stents into the intersection of a main vessel and a branch vessel such that a side opening in the main stent is positioned in registry with the ostium of the branch vessel, and such that the branch stent extends through the side opening in the main stent and into the branch vessel. This can be accomplished by deploying a main stent within the main vessel such that a side opening in the main stent is in registry with the ostium of the branch vessel with a second guidewire passing out through the side opening in the main stent and into the branch vessel, as described above. A branch stent is then subsequently advanced over the second guidewire and into the branch vessel such that the branch stent passes out through the side opening in the main stent and into the branch vessel. (A separate deployment catheter, or alternatively, the second catheter itself may be used for positioning and deploying the branch stent in the branch vessel). The main stent may optionally include radially expandable portions which protrude outwardly from the side opening in the main stent and into the walls of the branch vessel, holding the side opening in registry with the ostium of the branch vessel.

The side opening in the main stent can be positioned in alignment with the ostium of the branch vessel by viewing relative movement of radiopaque markers positioned on each of the main catheter and the flexible side sheath (or second catheter). The relative marker movement indicates that the distal portion of the flexible side sheath (or second catheter) which is positioned adjacent the side opening in the main stent is advancing into the ostium of the branch vessel, thereby indicating the position of the side opening of the main stent with respect to the ostium of the branch vessel. The flexible side sheath (or second catheter) will deflect into the branch vessel as it is advanced over the second guidewire, (while the main catheter itself moves distally along through the main vessel over the first guidewire).

Such relative movement of the radiopaque markers may be viewed as a rotation of a marker positioned on the flexible side sheath with respect to a marker(s) positioned on the main catheter, or as a separation between the marker on the flexible side sheath (or second catheter) with respect to a marker(s) on the main catheter. According to the invention the marker on the flexible side sheath (or second catheter) is positioned adjacent a marker on the main catheter, such that the relative marker motion will be viewable in an image as a separation occurring between the two markers. In addition, markers may be positioned at the distal ends of the flexible side sheath (or second catheter) and the main catheter, such that the separation between these markers will be relatively larger, and thus can be easily viewed. Moreover, when the markers are positioned at the distal ends of the main stent and side member, the surgeon will view the separation of these markers earlier than would be the case if these markers were disposed at a more proximal location.

In addition, a plurality of markers may be positioned on the catheter with a marker positioned at locations corresponding to each of the proximal and distal ends of the main stent. A medial marker may also be included, positioned halfway between the distal and proximal markers, for indicating the position of the side hole in the main stent, (which is preferably positioned halfway between the distal and proximal ends of the stent).

The relative movement of the markers positioned on the catheter and those positioned on the flexible side sheath (or second catheter) can be observed fluoroscopically as the markers are radiopaque and are preferably made of suitable materials including tungsten and gold.

Using the delivery system of the present invention, the main stent is deployed in the main vessel, (such as by an inflatable balloon at the distal end of the main catheter). Thereafter, a branch stent may be advanced through the at least partially deployed main stent and positioned in the branch vessel. The branch stent is advanced through the at least partially deployed main stent by a catheter which then deploys the branch stent in the branch vessel, (such as by an inflatable balloon at the distal end of the deployment catheter).

In an example (in which the flexible side sheath is slidably movable with respect to the main catheter) the branch stent can be deployed by first removing the side sheath from the second lumen of the dual lumen catheter, thereby leaving the second guidewire in position in the branch vessel. Thereafter, the deployment catheter can then be advanced over the second guide wire with its distal end extending into the branch vessel. A balloon disposed on the distal end of this deployment catheter can then be used to deploy the branch stent within the branch vessel. Alternately, the entire catheter system (comprising both the main catheter and the slidably attached side sheath) can be removed leaving the two guidewires in place such that the deployment catheter can be advanced over the second guidewire and into the branch vessel.

In an example (in which the flexible side sheath is not slidably movable with respect to the main catheter) the branch stent may be deployed by removing the entire system, (comprising both the main catheter and attached flexible side sheath), leaving the two guidewires in place such that the deployment catheter can then be advanced over the second guidewire and into the branch vessel. As such, the second catheter can then be advanced over the second guide wire with its distal end extending into the branch vessel.

Alternatively the first and second guidewires may first be positioned in the respective main and branch vessels. Thereafter, the main catheter can be advanced over the first guidewire and the flexible side sheath can simultaneously be advanced over the second guidewire. As such, a surgeon viewing movement between markers on the main catheter and the flexible side sheath can see exactly when and where the flexible side sheath enters the branch vessel.

The branch stent may optionally comprise a contact portion at its proximal end to secure the proximal end of the branch stent to the interior of the main stent through the side opening in the main stent.

In an example the distal end of the side sheath is positioned at the intersection of the main vessel and the branch vessel by viewing its position under fluoroscopy. Also, the second guidewire is inserted through the side sheath (or second catheter) and into the branch vessel under fluoroscopic viewing

An apparatus for aligning a side opening in a main stent in registry with the ostium of the branch vessel is described. A dual lumen catheter system in which a first guidewire is slidably received within a first lumen of the main catheter and a side sheath (or alternatively, the second catheter) is slidably received within the second lumen of this dual lumen catheter is described. A second guidewire is slidably received within the lumen of the side sheath. A main catheter with an attached flexible side sheath (or second catheter) is described.

To assist in guiding the second guidewire into the branch vessel, the side sheath (or second catheter) may taper to a narrow distal end, which may be curved slightly outwardly and which may comprise tungsten or other suitable radiopaque material such that it may be fluoroscopically viewed. A first balloon can be disposed over the first guidewire at a distal end of the dual lumen catheter and a second balloon is disposed over the second guidewire at a distal end of the second catheter (ie: the deployment catheter) which can be received within the second lumen of the dual lumen catheter.

An advantage of the present stent delivery system is that it avoids having to separately position first and second guidewires within the respective main and branch vessels prior to deployment of the main and branch stents thereover. Rather, only a single guidewire needs to initially be placed within the main vessel, with the delivery system subsequently deploying both the main and branch stents thereover.

The main stent may optionally include outwardly expandable portions which can be expanded from an initial position which is flush with the cylindrical body of the stent to protrude outwardly from the side opening in the main stent, thereby anchoring into the walls of the branch vessel, holding the side opening in registry with the ostium of the branch vessel. In an exemplary aspect, the cylindrical body of the main stent has an even surface, with an expandable portion positioned within the side opening of the cylindrical body, such that it is flush with the cylindrical body prior to expansion.

In addition, the branch stent may optionally comprise a contacting portion at its proximal end to secure the proximal end of the branch stent to the side opening in the main stent. In an exemplary aspect, the contacting portion comprises a flared proximal end.

Applications of the present system include the cardiac, coronary, renal, peripheral vascular, gastrointestinal, pulmonary, urinary and neurovascular systems and the brain. Further advantages of the present stent delivery system are that it provides an improved stent delivery apparatus, which may deliver main and branch stents to: 1) completely cover the bifurcation point of bifurcation vessels; 2) be used to treat lesions in one branch of a bifurcation while preserving access to the other branch for future treatment; 3) allow for differential sizing of the stents in a bifurcated stent apparatus even after a main stent is implanted; 4) treat bifurcation lesions in a bifurcated vessel where the branch vessel extends from the side of the main vessel; and 5) be marked with, or at least partly constructed of, material which is imageable by commonly used intraluminal catheterization visualization techniques including but not limited to ultrasound or x-ray.

As described herein, a side hole in the main stent refers to a relatively large hole which is intended to be aligned with the ostium of the branch vessel, as opposed to being any of the multiple passageways through the side of the main stent between respective struts in the stent geometry. Accordingly, the side hole in the main stent is a hole which is understood to be larger than other passages through the stent. In certain aspects, this side hole is defined by a band of continuous material which defines the perimeter of the side hole. This continuous band of material comprises discontinuities over its length so that the area of the side hole expands together with the expansion of the stent. In various aspects, the continuous band comprises protrusions which project inwardly from a peripheral edge of the side opening. Possibly, these protrusions (or expandable portions) are initially aligned within a cylindrical envelope of the tubular body of the main stent. The invention is defined by appended claim 1 with preferred embodiments defined in the dependent claims.

### BRIEF DESCRIPTION OF THE DRAWINGS

Fig 1. is an illustration of a dual lumen catheter with a main catheter and an attached slidably movable side sheath, the side sheath being slidably received within a second lumen of the dual lumen catheter.
Fig 2. is an illustration of a dual lumen catheter having similar to Fig. 1, but having a second catheter slidably received within a second lumen of the dual lumen catheter, with balloons positioned on the distal ends of both of the main catheter and the second catheter.
Fig 3 is an illustration of a second aspect of the present stent delivery system, showing a main catheter with a flexible side sheath attached thereto.
Fig 4 is a close up illustration of the distal end of the stent delivery system of Fig. 3 with a main stent positioned thereon.
Fig. 5 is a sectional side elevation view corresponding Fig. 4, showing the markers in alignment, prior to entering the branch vessel.
Fig. 6 is an illustration of a placement of first guidewire within a main vessel.
Fig. 7 is an illustration of the main catheter and side sheath of Fig. 1 advanced over the first guidewire to a position where the side opening in the main stent is adjacent with the ostium of the branch vessel.
Fig. 8A is an illustration corresponding to Fig. 7, but with the second guidewire advanced out of the distal end of the side sheath, through the side opening in the main stent and into the branch vessel.
Fig. 8B corresponds to Fig. 8A, but with the flexible side sheath removed.
Fig. 9 is an illustration corresponding to Fig. 8B, but with the branch stent advanced over the second guidewire and through the side opening in the main stent and into the branch vessel by a second catheter received within the second lumen of the dual lumen catheter.
Fig. 10 is an illustration of the deployment of the branch stent of Fig. 9 by a second balloon disposed over the second guidewire.
Fig. 11 is an illustration of the catheter and attached flexible side sheath of Figs. 3 and 4 advanced over the first guidewire to a position near the ostium of the branch vessel.
Fig. 12 is an illustration corresponding to Fig. 11, but with the second guidewire advanced out of the distal end of the side sheath, through a side opening in a main stent and into the branch vessel.
Fig. 13 is an illustration corresponding to Fig. 12, but with the catheter and attached flexible side sheath advanced over the first and second guidewire such that the distal end of the flexible side sheath is deflected into the branch vessel, showing the separation between radiopaque markers on the catheter and flexible side sheath.
Fig. 14 is a sectional side elevation view corresponding Fig. 13.
Fig. 15 is an illustration corresponding to Fig. 14, but showing the main catheter and flexible side sheath removed with a branch stent (positioned on a deployment catheter) advanced over the second guidewire and through the side opening in the main stent and into the branch vessel.
Fig. 16 is an illustration corresponding to Fig. 14, but showing the branch stent (positioned on a deployment balloon attached to the flexible side sheath) advanced over the second guidewire and through the side opening in the main stent and into the branch vessel.
Fig. 17 is an illustration of the further deployment of the branch stent of Fig. 15 by a balloon disposed on a deployment catheter received over the second guidewire.
Fig. 18 is an illustration of the fully deployed main and branch stents with the guidewires and stent delivery system removed.
Fig. 19 shows an example with outwardly expandable portions disposed around the side opening on the main stent fully deployed.
Fig. 20 is a side elevation view of the system of Fig. 4, showing both proximal and distal ends of an example, showing exemplary dimensions..
Fig. 21 is another illustration of the distal end of the system shown in Figs. 4 and 5.,
Fig. 22 is an illustration of an aspect of the proximal end of an example.
Fig. 23 is an illustration of a kit comprising the present apparatus, and instructions for use.

### DESCRIPTION OF THE SPECIFIC EMBODIMENTS

The present invention provides systems for positioning first and second guidewires in respective main and branch vessels at a vessel bifurcation. In additional aspects, a system for aligning the side hole in a main stent in registry with the ostium of a branch vessel is provided. In further additional aspects, main and branch stents are deployed in the bifurcation over the first and second guidewires.

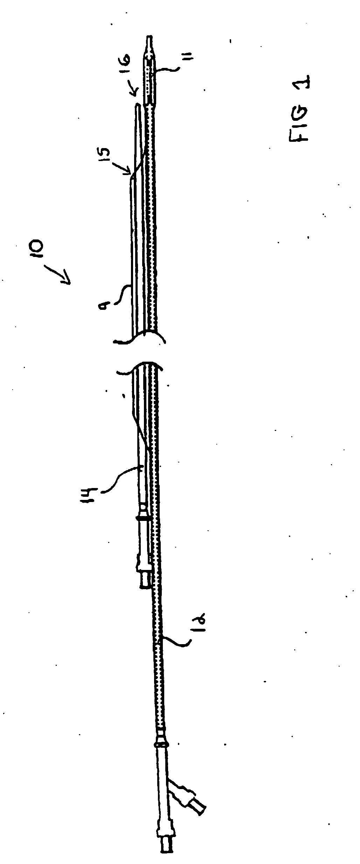

Figs. 1, 2 and 6 to 10 show a first aspect comprising a dual lumen catheter which comprises a main catheter and a flexible side sheath (or alternatively, a second catheter) which is slidably positionable with respect to the main catheter.

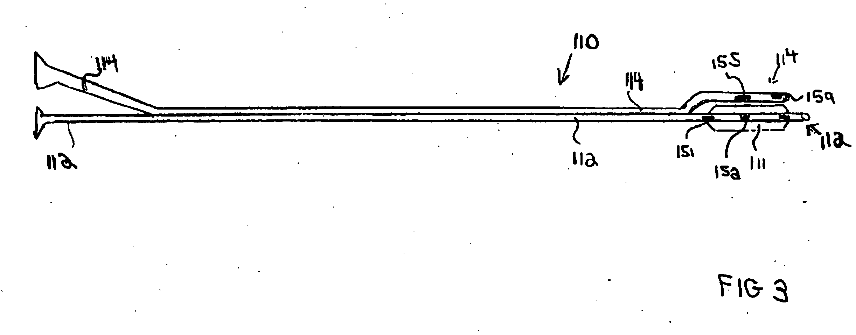

Figs. 3 to 5 and 11 to 17 and 21 show a second aspect comprising a main catheter with a flexible side sheath (or alternatively, a second catheter) which is attached to the main catheter.

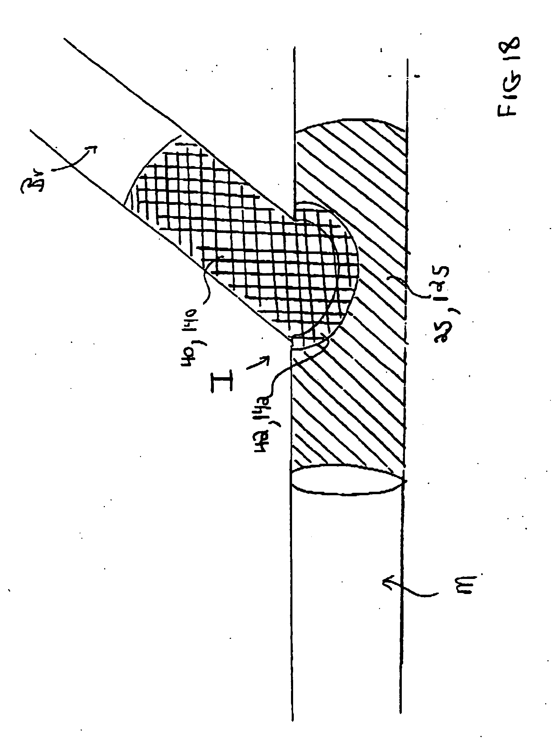

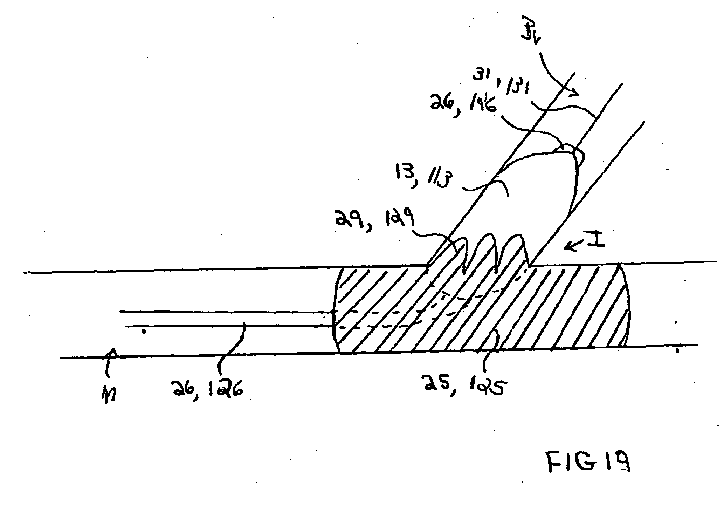

Fig. 18 shows a fully deployed main and branch stent which may be deployed, and Fig. 19 shows an example with outwardly expandable portions disposed around the side opening on the main stent fully deployed.

### Main Catheter with Slidably Positionable Side Sheath (Figs. 1,2 and 6 to 10):

The present catheter system comprises a dual lumen catheter having a guidewire received through its first lumen. In one aspect, a side sheath is slidably received through the second lumen of the dual lumen catheter. A second guidewire is received through the side sheath and the side sheath is slidably positionable (with respect to a main catheter) so as to optionally align a side hole in a main stent disposed on a main catheter with the ostium of a branch vessel.

In an alternative aspect, a second catheter is instead slidably received through the second lumen of the dual lumen catheter. The second catheter may optionally have a balloon disposed at its distal end such that first and second balloons are disposed over the first and second guidewires at the distal ends of the respective main and second catheters, for deploying main and branch stents respectively.

Referring to Fig. 1, a dual lumen catheter system 10 is provided comprising a main catheter 12 and a side sheath 14, wherein side sheath 14 is slidably received within lumen 15 which may be formed as an extending side portion 9 of catheter 12 such that side sheath 14 can be axially displaced with respect to catheter 12. The interior lumen 15 of extending side portion 9 is preferably formed to reduce sliding friction with side sheath 14. In an example, extending side portion 9 of catheter 12 forming lumen 15 is fabricated of pebax with graphite. Alternatively, the inner surface of lumen 15 may have metal powders, glass beads, Teflon powder or other inorganic fillers imbedded therein.

Side sheath 14 is flexible, (which aids in positioning its distal end to pass though a side hole in a main stent, as will be explained).

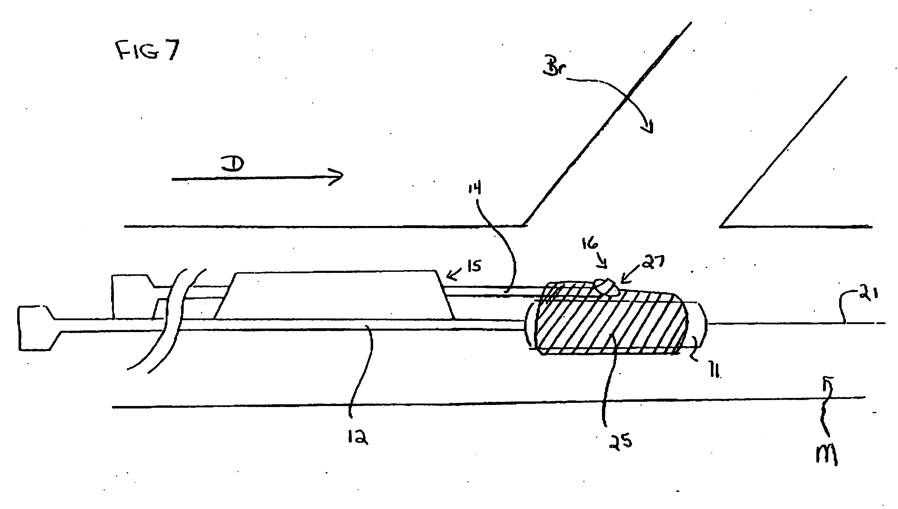

As will be explained with reference to Figs. 7 to 10, an advantage of the present dual lumen catheter 12 is that catheter 12 can be positioned at a desired location so as to align side hole 27 of main stent 25 at the ostium of a branch vessel by positioning the distal end 16 of side sheath 14 at the ostium of the branch vessel. Specifically dual lumen catheter 12 can be positioned proximal the intersection of the main and branch vessels to deploy a main stent 25 in the main vessel M with side sheath 14 positioned at the vessel intersection I to deploy a second guidewire 31 passing out through side hole 27 in main stent 25 and into branch vessel Br such that only one guidewire 21 needs to initially be positioned within main vessel M prior to subsequent deployment of main and branch stents 25 and 40.

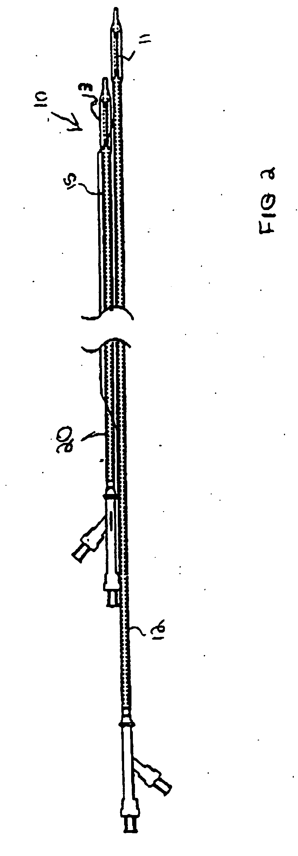

As can be seen in Fig. 2, a second catheter 20 can instead be slidably received in lumen 15 (in place of side sheath 14). A balloon 11 disposed at the distal end of dual lumen catheter 10 may be used to deploy a main stent in a main vessel. In this aspect, side sheath 14 may preferably be first removed (ie: withdrawn over guidewire 31) after guide wire 31 has been positioned in the branch vessel. Alternatively, guidewire 31 may instead be positioned in branch vessel Br by second catheter 20, such that side sheath 14 is not required. The second catheter 20 having a balloon 13 disposed at its distal end is then advanced over guide wire 31 with balloon 13 deploying an optional branch stent 40, as will be explained.

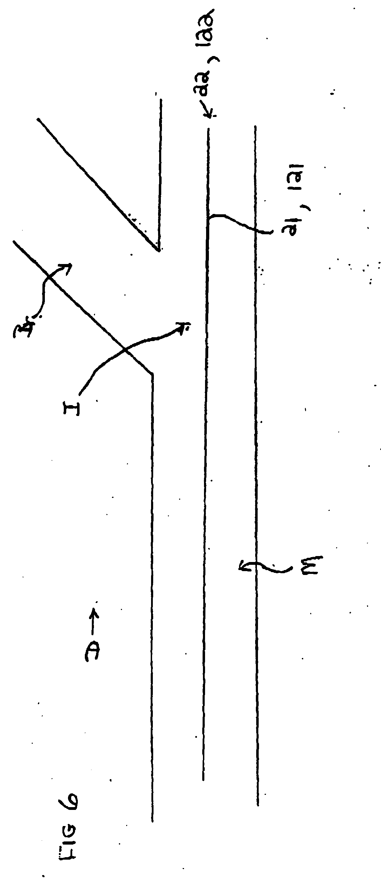

In a preferred aspect, the delivery system of the present invention is suitable for aligning a side hole in a main stent to a position in registry with the ostium of a branch vessel. This first aspect is illustrated in the sequential steps shown in Figs. 6 to 8.

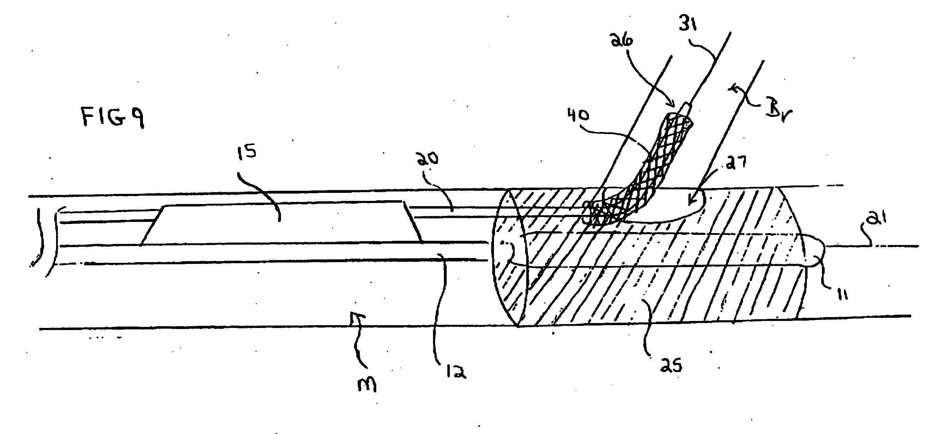

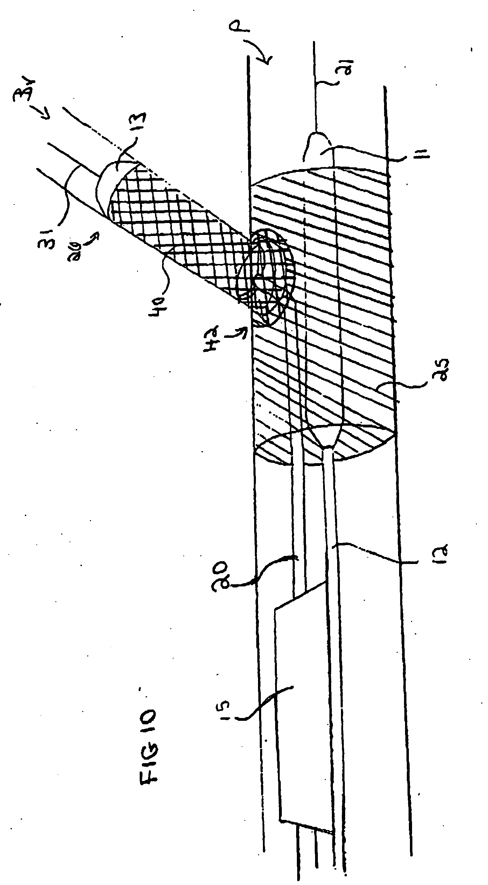

In another preferred aspect, the delivery system of the present invention is suitable for deploying a branch stent in a branch vessel with the branch stent extending into the branch vessel through a side opening in the main stent. This second aspect is illustrated in the sequential steps shown in Figs. 9 and 10. (The sequential steps shown in Figs. 9 and 10 are accomplished after the sequential steps shown in Figs. 6 to 8).

Referring first to Fig. 6, a first guidewire 21 is advanced through a main vessel M in direction D such that distal end 22 of guidewire 21 extends past the intersection of a main vessel M and a branch vessel Br.

As shown in Fig. 7, main catheter 12 is then advanced in direction D over guidewire 21 such that a main stent 25, (which is supported at a distal end of catheter 12, as shown), is oriented such that side hole 27 of stent 25 is positioned generally adjacent the ostium of branch vessel Br. (Guidewire 21 is received within a lumen in catheter 12.) As can be seen, stent 25 may be crimped down onto the distal end of side sheath 14, as shown. Optionally, distal end 16 of catheter 14 has tungsten, or other suitable radiopaque material, deposited thereon such that it can be viewed and positioned fluoroscopically. Optionally, stent 25 is initially crimped onto balloon 11 with distal end 16 of side sheath 14 projecting outwardly through side opening 27 as shown. As such, fluoroscopic positioning of distal end 16 aligns side opening 27 to branch vessel Br, as main catheter 12 supporting stent 25 and side sheath 14 initially move together when stent 25 is initially crimped onto balloon 11.

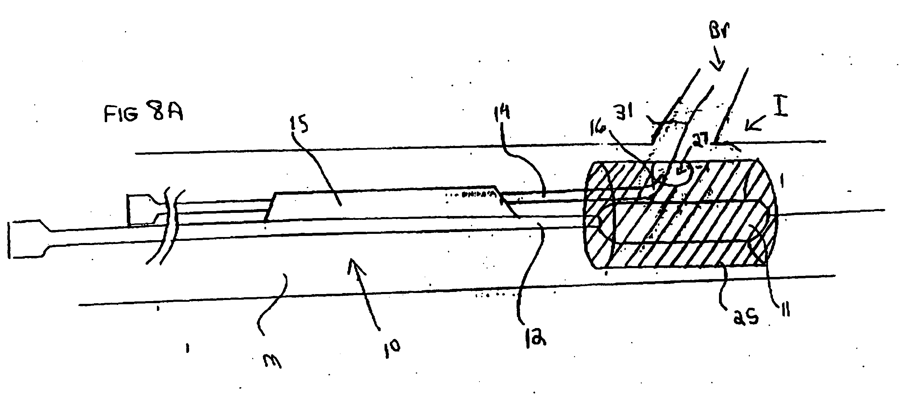

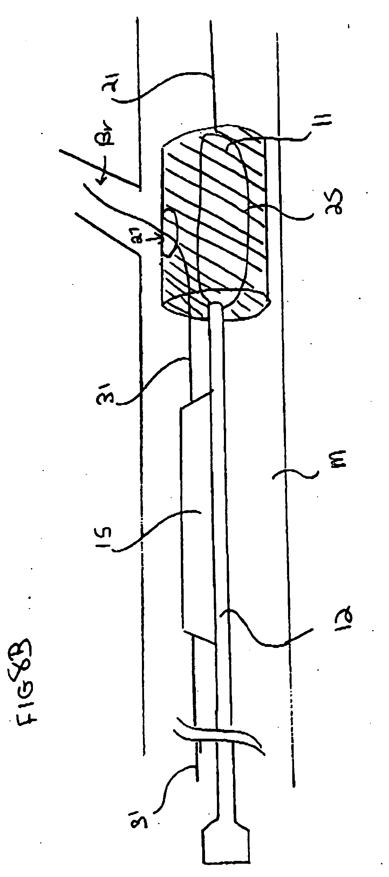

As shown in Fig. 8A, after distal end 16 of catheter 14 is positioned as shown, a second guidewire 31 can then be advanced out of distal end 16 of side sheath 14, passing through side opening 27 in stent 25 and into branch vessel Br. As also shown, balloon 11 may be partially or fully inflated, thereby expanding main stent 25 so as to provide an access space for a distal end 26 of second catheter 20 (Fig. 9) to be advanced therethrough, (after side sheath 14 has been removed as shown in Fog. 8B). Alternatively, second catheter 20 (as shown in Fig. 9) can instead be used in place of side sheath 14 to deploy guidewire 31, (avoiding the step shown in Fig. 8A and 8B). In one aspect, main stent 25 is at least partially deployed within main vessel M; and the distal end of second catheter 20 is then received within the at least partially deployed main stent 25.

In further optional aspects, additional sequential steps, as illustrated in Figs. 9 and 10, are carried out after the steps illustrated in Figs. 6 to 8B to deploy a branch stent in a branch vessel with the branch stent extending through a side opening in the main stent. Specifically, Fig. 9 is similar to Fig. 8B, but side sheath 14 has instead been replaced with second catheter 20 which has been advanced over guidewire 31. Branch stent 40 is disposed on the distal end 26 of catheter 20. As shown in Fig. 9, distal end 26 of catheter 20 can be advanced over guidewire 31 such that stent 40 is advanced through side hole 27 in stent 25 and is positioned in branch vessel Br. In various aspects, side sheath 14 is used to position guidewire 31, and is then removed and replaced by second catheter 20. In alternate aspects, second catheter 20 is instead used to position guidewire 31 itself.

As shown in Fig. 10, stent 40 can then be fully deployed within branch vessel Br by inflating second balloon 13 disposed at distal end 26 of catheter 20, Main stent 25 can be fully deployed by inflation of balloon 11 either before, after or concurrently with, branch stent 40 being deployed by inflation of balloon 13. As can also be seen in Fig. 18, stent 40 may further comprise a contact portion 42 which remains disposed within side opening 27 thereby securing the proximal end of stent 40 to side opening 27 of stent 25, thereby providing a bifurcated stent arrangement covering vessel intersection I.

Lastly, as can also be seen in Fig. 18, catheter system 10, (comprising catheter 12, side sheath 14 or catheter 20, and guidewires 21 and 31) can be removed after deployment of stents 25 and 40, leaving a bifurcated support at the intersection of main vessel P and branch vessel Br as shown.

As shown in the optional step of Fig. 19, balloon 13 on catheter 14 can then be inflated to deploy radially expandable portions 29 extending laterally outward from the edges of side opening 27, such that portions 29 are pushed against the walls of branch vessel Br, such that side opening 27 is positioned in registry with the ostium of branch vessel Br. Further description of such radially expandable portions 29 which extend laterally outward from the edges of side opening 27 is set forth in Published PCT Patent Application WO 99/00835, filed 01/14/98.

The delivery system of the present invention may be employed in a method of aligning a side opening 27 in a main stent 25 in registry with the ostium of a branch vessel Br, comprising: advancing a first guidewire 21 through a main vessel M such that a distal end of guidewire 21 extends past intersection I of main vessel M and the branch vessel Br; advancing main stent 25 over first guidewire 21 with a dual lumen catheter system 10, wherein first guidewire 21 is received within a first lumen of main catheter 12; positioning a side sheath 14 received through the second lumen of dual lumen catheter system 10 such that a distal end 16 of side sheath 14 is positioned at intersection I of main vessel M and branch vessel Br; and advancing second guidewire 31 through side sheath 14 and out through the side opening 27 in main stent 25 and into branch vessel Br, thereby aligning side opening 27 in main stent 25 with the ostium of branch vessel Br. In additional aspects, a branch stent 40 is advanced over second guidewire 31 and out through the side opening 27 in main stent 25 and into the branch vessel Br by second catheter 20.

In additional aspects, side sheath 14 is positioned through the second lumen of dual lumen catheter 20 such that distal end 16 of side sheath 14 is positioned at intersection I of main vessel M and branch vessel Br by viewing the position of the distal end of the second catheter 20 under fluoroscopy. In additional aspects, second guidewire 31 is advanced through side sheath 14 and into branch vessel Br by viewing the position of the distal end of second guidewire 31 under fluoroscopy.

In preferred aspects, a first balloon 11 is disposed at a distal end of the dual lumen catheter 10 over first guidewire 31; and a second balloon 13 is disposed at a distal end of second catheter 20 over second guidewire 31.

### Main Catheter with Attached Side Sheath (Figs. 3 to 5 and 11 to 17 and 21):

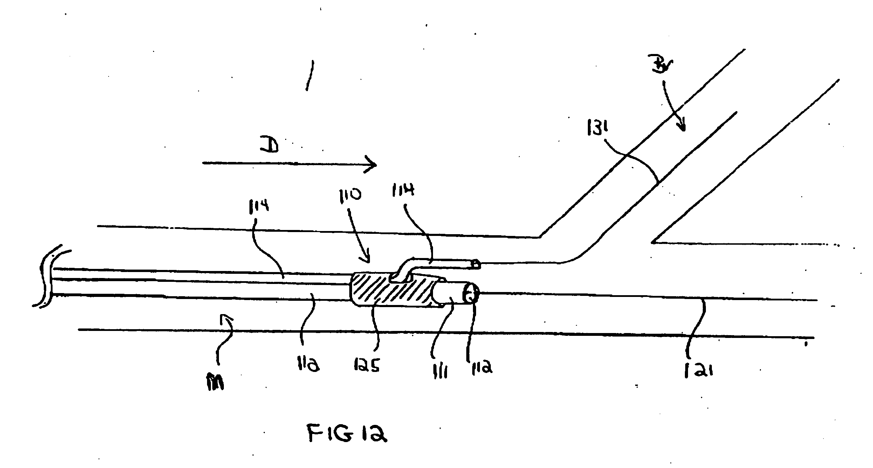

Referring to Figs. 3 to 5, the present stent delivery system 110 comprises a first catheter 112 having an attached flexible side sheath 114. An inflatable balloon 111 may optionally be positioned at the distal end of first catheter 112. As is shown in Figs. 12 to 14, first catheter 112 is receivable over a first guidewire 121 and flexible side sheath 114 is receivable over a second guidewire 131. The present stent delivery system 110 can be used to advantageously position first and second guidewires in respective main and branch vessels. In further aspects, the present stent delivery system 110 can be used to position main and branch stents in the main and branch vessels (over the respective main and branch guidewires).

In another aspect, guidewires 121 and 131 are pre-positioned in respective main and branch vessels and stent delivery system 110 is advanced simultaneously thereover into vessel intersection I. In this aspect, the location of the vessel intersection and the moment in time at which side sheath 114 enters branch vessel Br can be determined by fluoroscopically viewing relative marker band rotation or separation as will be explained.

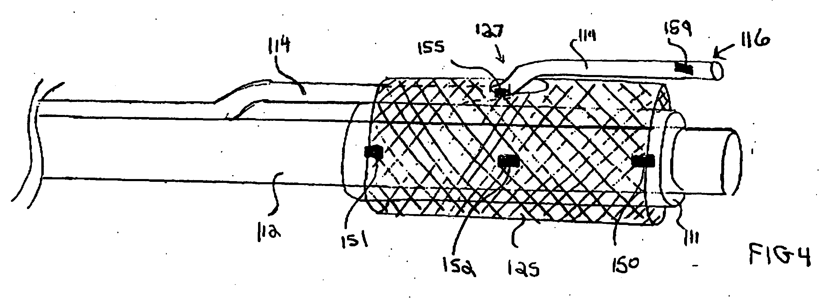

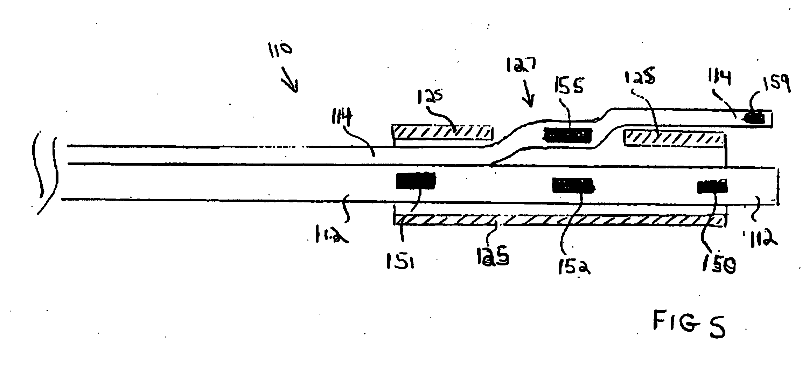

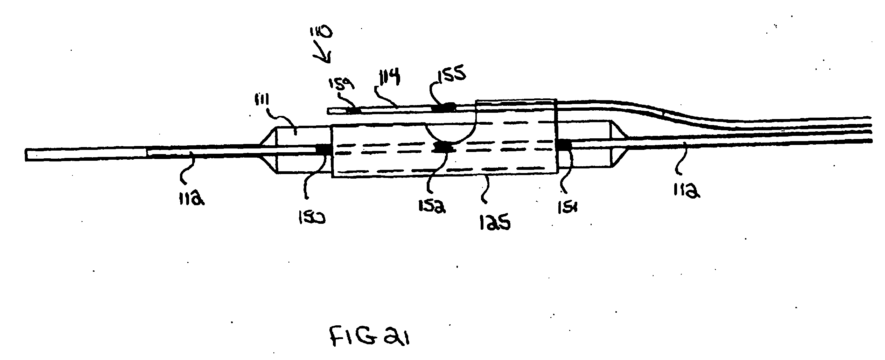

Referring to Fig. 4, stent 125 can be crimped down onto flexible side sheath 114, as shown. Preferably, stent 125 is initially crimped onto balloon 111 with distal end 116 of side sheath 114 projecting outwardly through side opening 127 as shown. Fig. 21 is another illustration of the distal end of the system shown in Figs. 4 and 5.

In the following, a method of positioning main stent 125 at a vessel bifurcation (intersection I) such that a side opening 127 in main stent 125 is positioned at the ostium of a branch vessel Br is described.

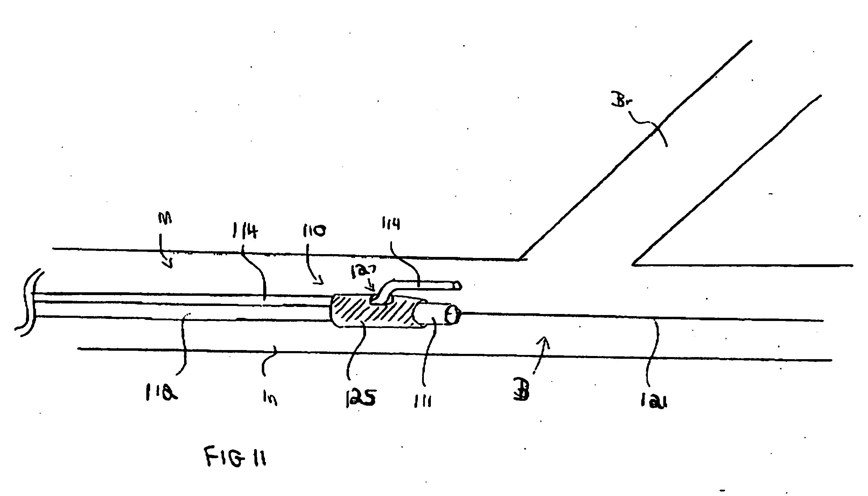

Referring to Fig. 6, a main guidewire 121 is first positioned in the main vessel M such that a distal end 122 of main guidewire 121 extends past vessel intersection I. Referring next to Fig. 11, stent delivery system 110 is then advanced to a position proximate vessel intersection I, wherein catheter 112 is received over first guidewire 121, and wherein main stent 125 is positioned over catheter 112 with flexible side sheath 114 positioned to pass through the interior of main stent 125 and out of side opening 127 in main stent 125, as shown. In one aspect, as shown in Fig. 12, second guidewire 131 is then advanced through flexible side sheath 114 attached to catheter 112 and into branch vessel Br. In an alternate aspect, guidewires 121 and 131 are pre-positioned in respective main and branch vessels M and Br, and system 110 is advanced thereover, with main catheter 112 passing over guidewire 121 and flexible side sheath 114 passing over guidewire 131.

In one aspect, side opening 127 in main stent 125 is positioned in alignment with the ostium of branch vessel Br simply by the presence of second guidewire 131 (and flexible side sheath 114) extending from an interior of main stent 125 out through side opening 127 in main stent 125 and into branch vessel Br. In this aspect, the insertion of a branch stent over guidewire 131 through side opening 127 in main stent 125 and into branch vessel Br serves to align the side opening 127 with the ostium of branch vessel Br.

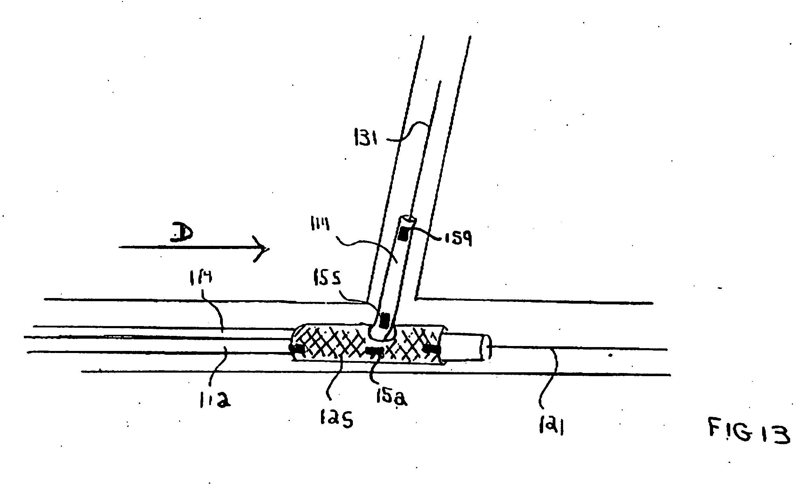

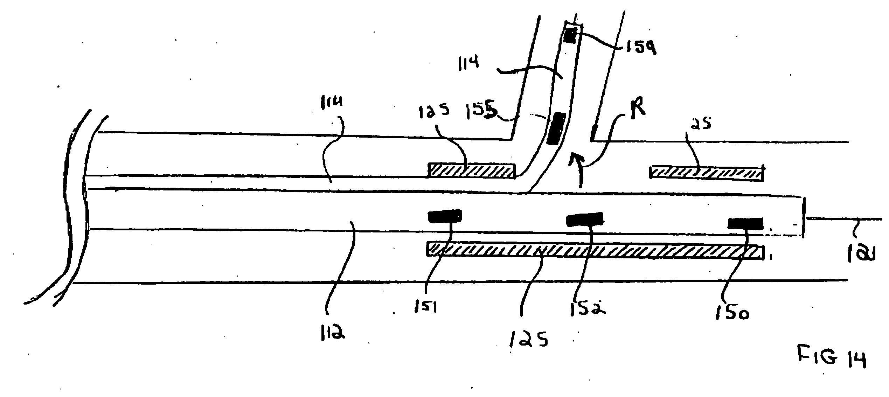

In another aspect, however, stent delivery system 110, (comprising catheter 112 and attached flexible side sheath 114), are subsequently advanced distally in direction D to the position as shown in Fig. 13 and 14, with catheter 112 being advanced over first guidewire 121 while flexible side sheath 114 is advanced over second guidewire 131. In this aspect, an operator views relative movement between a radiopaque marker positioned on the flexible side sheath with respect to at least one radiopaque marker positioned on the catheter, wherein the relative marker movement indicates that a portion of the flexible side sheath adjacent the side opening in the main stent is advancing into the ostium of the branch vessel, thereby indicating the position of side opening 127 of main stent 125 with respect to the ostium of branch vessel Br.

Specifically, referring to Figs. 5 and 14, a distal marker 150, a proximal marker 151 and a medial marker 152 may be disposed on catheter 112. The location of proximal marker 151 corresponds to the location of the proximal end of stent 125, the location of distal marker 150 corresponds to the location of the distal end of stent 125, and the location of medial marker 152 corresponds to the location of side opening 127 of stent 125. At least one marker 155 is positioned on flexible side sheath 114 as shown. Marker 155 is positioned adjacent to medial marker 152. In addition, a marker 159 may be positioned at the distal end of side sheath 114. As such, the separation between markers 159 and 150 can be observed from the point in time when the distal end of side sheath 114 initially starts to advance into branch vessel Br over second guidewire 131.

As can be seen by comparing Figs. 5 and 14, as stent delivery system 110 is advanced distally such that the distal end of flexible side sheath 114 is received in branch vessel Br, (Fig. 14), marker 155 will move in direction R relative to markers 150, 151 and 152. In particular, an increasing separation distance will occur between marker 155 positioned on flexible side sheath 114 and marker 152 positioned on catheter 112 as catheter 112 is advanced distally over first guidewire 121 while flexible side sheath 114 is simultaneously advanced distally over second guidewire 131.

In an additional aspect, each of markers 152 and 155 are slightly elongated and rectangle shaped, (as shown), such that relative rotational movement therebetween can also be observed. Markers 150,151,152, 155 and 159 may be made of tungsten or gold.

When the operator views the relative motion (rotation or increasing separation) between markers 152 and 155, this indicates that a portion of flexible side sheath 114 has entered the ostium of branch vessel Br. By viewing the position of markers 150,151 and 152, the operator can also determine the position of the distal and proximal ends of stent 25 and the position of side opening 127 with respect to the ostium of branch vessel Br.

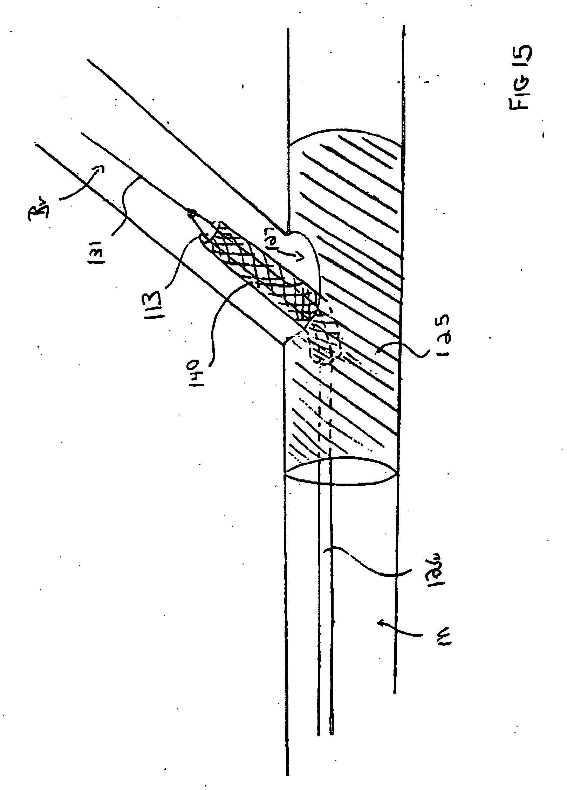

Systems for deploying a branch stent into branch vessel Br with main stent 125 positioned such that side opening 127 is in registry with the ostium of branch vessel Br are described. In these aspects, as illustrated in Figs. 15 through 19, branch stent 140 is advanced through the interior of main stent 125, passing through side opening 127 and into branch vessel Br.

Fig. 15 is an illustration of branch stent 140, (disposed on the distal end of a deployment catheter 126), being advanced over second guidewire 131, passing through side opening 127 in main stent 125 into branch vessel Br. As can be seen, in one aspect, main catheter 112 of stent delivery system 110 may be used to deploy main stent 125 and then system 110 may be removed. Thereafter, a second (deployment) catheter 126 can be advanced over second guidewire 131 to position stent 140 for deployment in the branch vessel.

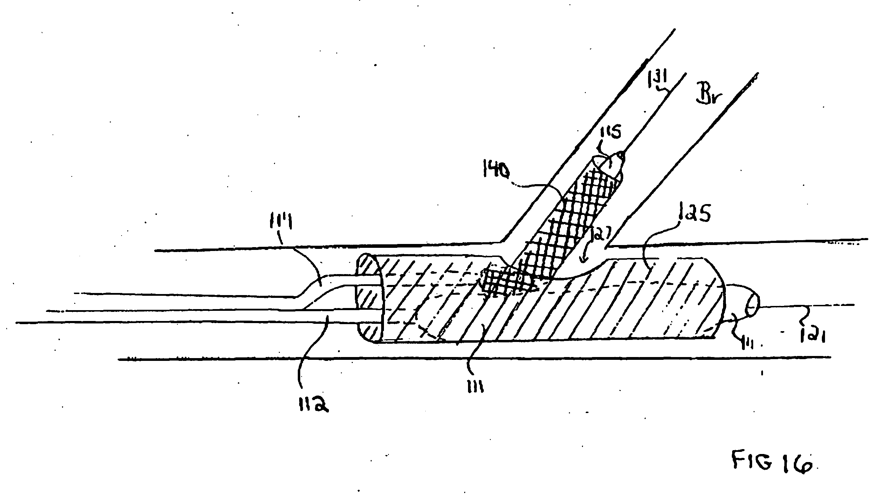

In an alternative aspect, as shown in Fig. 16, stent 125 may be partially deployed in main vessel M by balloon 111 and branch stent 140 may be partially deployed in branch vessel Br by an optional balloon 115 on the distal end of side sheath 114 which is advanced through the partially expanded interior of main stent 125, passing out through side opening 127 in main stent 125 while stent delivery system 110 remains adjacent vessel intersection I.

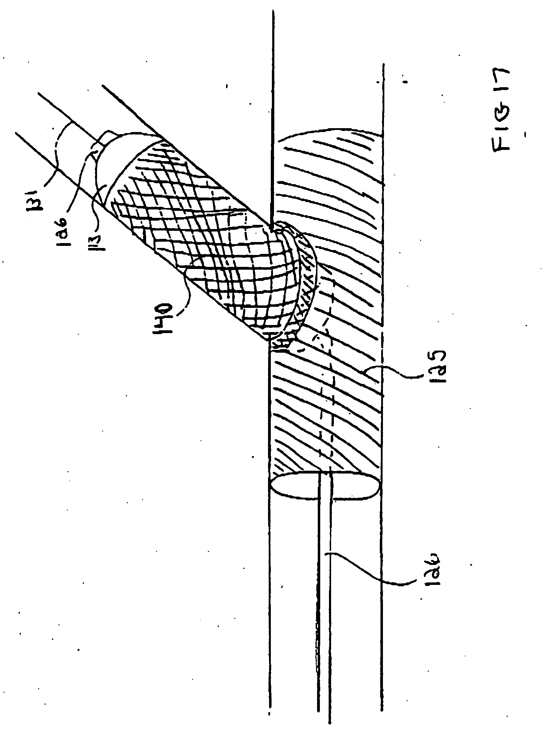

Fig. 17 is an illustration of the deployment of branch stent 140 by a balloon 113 disposed on the distal end of second catheter 126, which is itself received over second guidewire 131. In this aspect an inflatable balloon 113 disposed at the distal end of second catheter 126 is used to deploy branch stent 140.

Fig. 18 is an illustration of the fully deployed main and branch stents 125 and 140 with guidewires 121 and 131 and stent delivery system 110 removed. As can also be seen, stent 140 may further comprise a contact portion 142 which remains disposed within side opening 127 thereby securing the proximal end of stent 140 to side opening 127 of stent 125, thereby providing a bifurcated stent arrangement covering vessel intersection I. Such a contacting portion 142 is further described in co-pending PCT Patent Application WO 99/00835, filed 01/14/98.

Lastly, Fig. 19 shows an example with outwardly expandable portions disposed around the side opening on the main stent. Specifically, balloon 113 on catheter 126 can also be inflated to deploy radially expandable portions 129 which extend laterally outward from an initial position flush with the cylindrical body of stent 125 to a position where portions 129, (disposed around the edges of side opening 127), are anchored against the walls of branch vessel Br, such that side opening 127 is positioned in registry with the ostium of branch vessel Br. Further description of such radially expandable portions 129 which extend laterally outward from the edges of side opening 127 is set forth in Published PCT Patent Application No. WO 99/00835 filed 01/14/98.

The delivery system of the present invention may be used in kits comprising the delivery system of the present invention and instructions for use setting forth any of the methods for use described herein.

The delivery system of the present invention may also be employed in a method of positioning a main stent 125 at a vessel bifurcation (intersection I) such that a side opening 127 in main stent 125 is positioned at the ostium of branch vessel Br, comprising: positioning a main guidewire 121 in the main vessel M such that a distal end of the main guidewire 121 extends past the bifurcation; advancing a stent delivery system 110 to a position proximate the bifurcation I, the stent delivery system 110 comprising a catheter 112 with a flexible side sheath 114 attached thereto, wherein catheter 112 is received over main guidewire 121, and wherein the main stent 125 is positioned over catheter 112 with the flexible side sheath 114 positioned to pass through the interior of main stent 125 and out of side opening 127 in main stent 125; advancing a branch guidewire 131 through flexible side sheath 114 attached to catheter 112 and into branch vessel Br; and subsequently, advancing catheter 112 over the main guidewire 121 while advancing flexible side sheath 114 over branch guidewire 131 while viewing relative movement of a marker 155 or 159 positioned on the flexible side sheath with respect to at least one marker 151, 152, 150 positioned on catheter 112, wherein the relative movement indicates that a portion of flexible side sheath 114 adjacent side opening 127 in main stent 125 is advancing into the ostium of branch vessel Br, thereby indicating the position of side opening 127 of main stent 125 with respect to the ostium of branch vessel Br.

In various aspects, viewing relative movement of a marker 155 or 159 positioned on flexible side sheath 114 with respect to at least one marker 151, 152, 150 positioned on catheter 112, comprises: viewing an increasing separation distance between marker(s) 155, 159 positioned on flexible side sheath 114 with respect to at least one marker 150, 151, 152 positioned on catheter 112 as catheter 112 is advanced over first guidewire 121 while flexible side sheath 114 is simultaneously advanced over second guidewire 131.

\/iewing the at least one marker positioned on the catheter may comprise viewing markers positioned adjacent the distal and proximal ends of the main stent.

The distal end of a second catheter 126 may be advanced over branch guidewire 131 and into branch vessel Br. A branch stent 140 may then be deployed within branch vessel Br, wherein branch stent 140 is positioned on the distal end of second catheter 126.

The delivery system of the present invention may be employed in a method of positioning a main stent 125 at a vessel bifurcation (intersection I) such that a side opening 127 in main stent 125 is positioned at the ostium of branch vessel Br, comprising: positioning a main guidewire 121 in the main vessel M such that a distal end of main guidewire 121 extends past the bifurcation; advancing a stent delivery system 110 to a position proximate the bifurcation, the stent delivery system comprising a catheter 112 with a flexible side sheath 114 attached thereto, wherein catheter 112 is received over main guidewire 121, and wherein main stent 125 is positioned over catheter 112 with flexible side sheath 114 positioned to pass through the interior of main stent 125 and out of side opening 127 in main stent 125; advancing branch guidewire 131 through flexible side sheath 114 and into branch vessel Br; and subsequently, advancing catheter 112 over main guidewire 121 while advancing flexible side sheath 114 over branch guidewire 131 such that side opening 127 in main stent 125 is positioned at the ostium of branch vessel Br.

A stent delivery system 110 is described for positioning a main stent 125 at a vessel bifurcation I such that side opening 127 in main stent 125 is positioned at the ostium of branch vessel Br, comprising: first guidewire 121; catheter 112 receivable over first guidewire 121, catheter 112 having at least one marker 150, 151, 152 positioned thereon; a flexible side sheath 114 attached to catheter 112, flexible side sheath 114 having at least one marker 155, 159 positioned thereon; and a second guidewire 131 receivable through the flexible side sheath.

It may further comprise a main stent 125 positioned on catheter 112, and at least one marker 155, 159 positioned on delivery system 110, preferably comprising distal and proximal markers 150 and 151. A stent delivery system 110 is described for positioning a main stent 125 at a vessel bifurcation I such that a side opening 127 in main stent 125 is positioned at the ostium of branch vessel Br, comprising: a catheter 112 having at least one marker 150, 151, 152 positioned thereon; and a flexible side sheath 114 attached to catheter 112, flexible side sheath 114 having at least one marker 155, 159 positioned thereon.

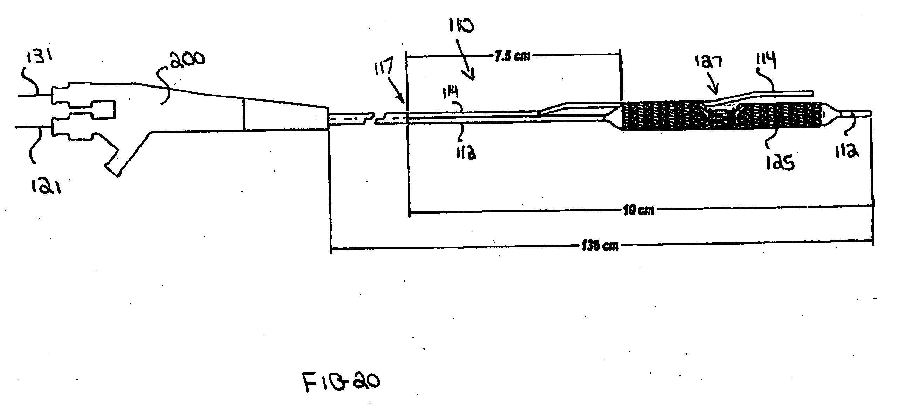

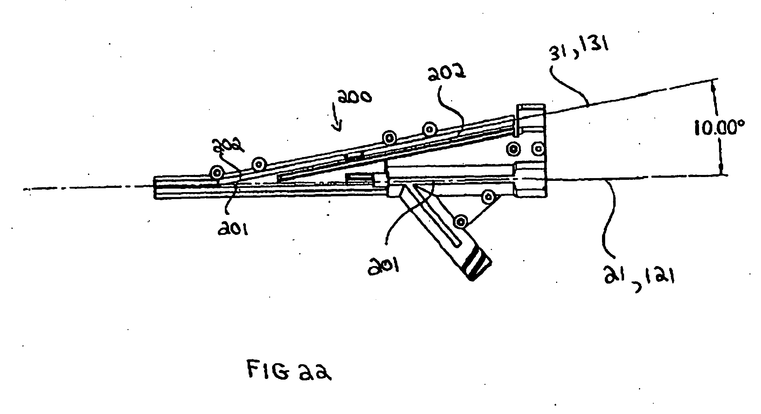

### Proximal End of Guidewire Delivery System (Figs. 20 and 22):

A mechanism may be disposed at the proximal end of present invention, for simultaneously handling respective main and branch guidewires. Referring to Fig. 20, a guidewire hub 200 is adapted with channels 201 and 202 passing therethrough in which guidewires 121 and 131 are passed, as shown. Alternatively, guidewire hub 200 can instead be used to receive guidewires 21 and 31 therethrough. The dimensions shown in Fig. 20 are understood to be exemplary, not limiting. As can be seen in Fig. 20, side sheath 114 and main catheter 112 separate from one another at point 117, which may be approximately 10 cm from their respective distal ends. Such a length of separation has been founded to be advantageous when rotating system 110 as it is being advanced distally in a patient.

As can be seen in Fig. 22, guidewires 21 and 31 (or 121 and 131) are received through guidewire hub 200 such that the guidewires are positioned at an angle to one another. This angle ranges from 0° to 20°. In another aspect, this angle is less than or equal to 10°. An advantage of angling the guidewires with respect to one another in this fashion, at these preferred angles, is that excessive friction is avoided when positioning or moving the guidewires. As such, simultaneous handling of the wires is facilitated. In addition, the guidewires are held close enough together such that an operator is able to simultaneously grasp and hold onto both guidewires with one hand while withdrawing the system over the two wires with the other hand. In addition, the guidewires are held far enough apart such that separate syringes can access each of channels 201 and 202, or separate luer fittings can be positioned to cover each of the proximal ends of channels 201 and 202.

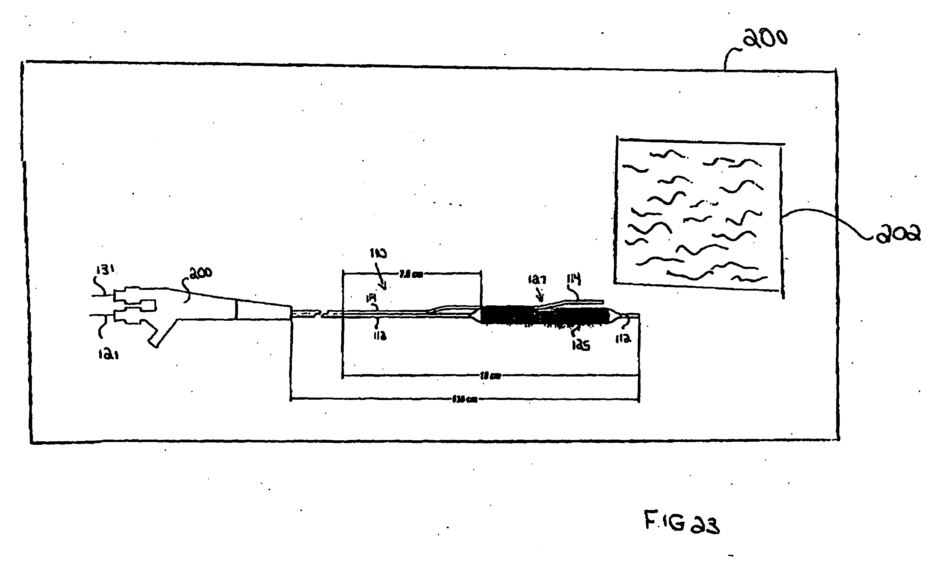

The delivery system of the present invention further may be used in kits comprising: any of the delivery systems set forth herein, in combination with instructions for use setting forth any of the methods described herein. Referring to Fig. 23, a kit 200, comprising a catheter system 110 (or alternately 10) and instructions for use 202 is also provided. Instructions for use 202 may comprise instructions for use comprising any of the methods of use described herein, including methods for deploying a main stent such that a side hole in the main stent is positioned in registry with the ostium of a branch vessel. Instructions for use 202 may be in written or machine readable form, as desired.

## Claims

1. A delivery system (110) for positioning at a vessel bifurcation, the system comprising:
a catheter (112) having a distal marker (150) and a proximal marker (151) positioned thereon; and
a flexible side sheath (114) positioned alongside said catheter (112), the flexible side sheath (114) having a side sheath marker (155,159) positioned thereon,
wherein said distal and proximal markers (150,151) are positioned relative to said side sheath marker (155,159) such that upon positioning of the flexible side sheath (114) at the vessel bifurcation, said flexible side sheath (114) is positioned and configured to enter a branch vessel, thereby separating said distal and proximal catheter markers (150,151) and said side sheath marker (155,159).

2. The delivery system (110) of claim 1, further comprising a stent (125), wherein the catheter (112) is positioned through the stent (125).

3. The delivery system (110) of claim 1, wherein said catheter further comprises a middle marker (152) positioned between said distal marker (150) and proximal marker (151).

4. The delivery system (110) according to any one of the preceding claims, further comprising a balloon positioned at a distal end of the catheter.

5. The delivery system (110) according to claim 1, wherein at least one of said catheter marker and said side sheath marker is radiopaque.

6. The delivery system (110) of claim 5, wherein at least one radiopaque marker comprises at least one of gold and tungsten.

7. The delivery system (110) of claim 3, wherein said side sheath marker (155) is substantially adjacent to said middle marker (152) before separating the markers on entry to the branch vessel.

8. The delivery system (110) of claim 2, wherein said side sheath (114) is attached to said catheter (112) at a first end of said side sheath and is positioned through a side opening (127) of the stent (125) at a second end of said side sheath.

9. The delivery system (110) of claim 1, wherein one of the distal marker (150) and the proximal marker (151) are positioned substantially adjacent the side sheath marker (155) before separating the markers on entry to the branch vessel.

10. The delivery system (110) of claim 2, wherein said stent (125) has a side opening (127) configured to be positioned at an ostium of a branch vessel and the flexible side sheath (114) is positioned through the stent (125) such that a distal end of the flexible side sheath projects through said side opening.

11. The delivery system (110) of claim 1, further comprising:
a first guidewire receivable through said catheter (112); and
a second guidewire receivable through said flexible side sheath (114).

12. The delivery system (110) of claim 1, wherein the side sheath marker (155,159) includes a first side sheath marker (155) and a second side sheath marker (159).

## Patentansprüche

1. Zuführsystem (110) zum Positionieren an einer Gefäßverzweigung, wobei das System aufweist:
einen Katheter (112), an dem ein distaler Marker (150) und ein proximaler Marker (151) positioniert sind; und
eine flexible Seitenhülse (114), die längsseits des Katheters (112) positioniert ist, wobei an der flexiblen Seitenhülse (114) ein Seitenhülsenmarker (155, 159) positioniert ist,
wobei der distale und der proximale Marker (150, 151) relativ zu dem Seitenhülsenmarker (155, 159) so positioniert sind, dass nach Positionierung der flexiblen Seitenhülse (114) an der Gefäßverzweigung die flexible Seitenhülse (114) entsprechend positioniert und konfiguriert ist, um in ein Zweiggefäß einzudringen, wodurch der distale und der proximale Kathetermarker (150, 151) und der Seitenhülsenmarker (155, 159) getrennt werden.

2. Zuführsystem (110) nach Anspruch 1, ferner mit einem Stent (125), wobei der Katheter (112) durch den Stent (125) positioniert wird.

3. Zuführsystem (110) nach Anspruch 1, wobei der Katheter ferner einen mittleren Marker (152) aufweist, der zwischen dem distalen Marker (150) und dem proximalen Marker (151) positioniert ist.

4. Zuführsystem (110) nach einem der vorhergehenden Ansprüche, ferner mit einem Ballon, der an einem distalen Ende des Katheters positioniert ist.

5. Zuführsystem (110) nach Anspruch 1, wobei der Kathetermarker und/oder der Seitenhülsenmarker strahlenundurchlässig ist.

6. Zuführsystem (110) nach Anspruch 5, wobei zumindest ein strahlenundurchlässiger Marker Gold und/oder Wolfram aufweist.

7. Zuführsystem (110) nach Anspruch 3, wobei der Seitenhülsenmarker (155) mit dem mittleren Marker (152) im Wesentlichen benachbart ist, bevor die Marker beim Eindringen in das Zweiggefäß getrennt werden.

8. Zuführsystem (110) nach Anspruch 2, wobei die Seitenhülse (114) an einem ersten Ende der Seitenhülse an dem Katheter (112) angebracht und durch eine Seitenöffnung (127) des Stents (125) an einem zweiten Ende der Seitenhülse positioniert ist.

9. Zuführsystem (110) nach Anspruch 1, wobei der distale Marker (150) oder der proximale Marker (151) mit dem Seitenhülsenmarker (155) im Wesentlichen benachbart positioniert ist, bevor die Marker beim Eindringen in das Zweiggefäß getrennt werden.

10. Zuführsystem (110) nach Anspruch 2, wobei der Stent (125) eine Seitenöffnung (127) hat, die entsprechend konfiguriert ist, um in einem Ostium eines Zweiggefäßes positioniert zu werden, und die flexible Seitenhülse (114) durch den Stent (125) so positioniert wird, dass ein distales Ende der flexiblen Seitenhülse durch die Seitenöffnung vorsteht.

11. Zuführsystem (110) nach Anspruch 1, ferner mit:
einem ersten Führungsdraht, der durch den Katheter (112) aufgenommen werden kann; und
einem zweiten Führungsdraht, der durch die flexible Seitenhülse (114) aufgenommen werden kann.

12. Zuführsystem (110) nach Anspruch 1, wobei der Seitenhülsenmarker (155, 159) einen ersten Seitenhülsenmarker (155) und einen zweiten Seitenhülsenmarker (159) aufweist.

## Revendications

1. Système de distribution (110) de positionnement à une bifurcation de vaisseau, le système comprenant :
un cathéter (112) possédant un marqueur distal (150) et un marqueur proximal (151) sur ledit cathéter ;
une gaine latérale flexible (114) disposée le long dudit cathéter (112), la gaine latérale flexible (114) possédant un marqueur de gaine latérale (155, 159) sur ladite gaine ;
dans lequel lesdits marqueurs distal et proximal (150, 151) sont disposés, par rapport audit marqueur de gaine latérale (155, 159) de telle sorte que, lors du positionnement de la gaine latérale flexible (114) à la bifurcation de vaisseau, ladite gaine latérale flexible (114) est positionnée et configurée pour pénétrer dans un vaisseau secondaire, pour ainsi séparer lesdits marqueurs de cathéter distal et proximal (150, 151) et ledit marqueur de gaine latérale (155, 159).

2. Système de distribution (110) selon la revendication 1, comprenant en outre une endoprothèse vasculaire (125), le cathéter (112) étant positionné à travers l'endoprothèse vasculaire (125).

3. Système de distribution (110) selon la revendication 1, dans lequel ledit cathéter comprend en outre un marqueur médian (152) disposé entre ledit marqueur distal (150) et ledit marqueur proximal (151).

4. Système de distribution (110) selon l'une quelconque des revendications précédentes, comprenant en outre un ballonnet disposé à une extrémité distale du cathéter.

5. Système de distribution (110) selon la revendication 1, dans lequel au moins un desdits marqueurs parmi le marqueur de cathéter et le marqueur de gaine latérale est opaque aux radiations.

6. Système de distribution (110) selon la revendication 5, dans lequel au moins un marqueur opaque aux radiations comprend au moins, soit de l'or, sur du tungstène.

7. Système de distribution (110) selon la revendication 3, dans lequel ledit marqueur de gaine latérale (155) est essentiellement adjacent audit marqueur médian (152) avant la séparation des marqueurs à l'entrée du vaisseau secondaire.

8. Système de distribution (110) selon la revendication 2, dans lequel ladite gaine latérale (114) est fixée audit cathéter (112) à une première extrémité de ladite gaine latérale et vient se disposer à travers une ouverture latérale (127) de l'endoprothèse vasculaire (125) à une deuxième extrémité de ladite gaine latérale.

9. Système de distribution (110) selon la revendication 1, dans lequel un des marqueurs parmi le marqueur distal (150) et le marqueur proximal (151) est disposé essentiellement en position adjacente au marqueur de gaine latérale (155) avant la séparation des marqueurs à l'entrée du vaisseau secondaire.

10. Système de distribution (110) selon la revendication 2, dans lequel ladite endoprothèse vasculaire (125) possède une ouverture latérale (127) configurée pour venir se disposer à un ostium d'un vaisseau secondaire, et la gaine latérale flexible (114) vient se disposer à travers l'endoprothèse vasculaire (125), de telle sorte qu'une extrémité distale de la gaine latérale flexible fait saillie à travers ladite ouverture latéral.

11. Système de distribution (110) selon la revendication 1, comprenant en outre:
un premier fil guide apte à être reçu à travers ledit cathéter (112) ; et
un deuxième fil guide apte à être reçu à travers ladite gaine latérale flexible (114).

12. Système de distribution (110) selon la revendication 1, dans lequel le marqueur de gaine latérale (155, 159) englobe un premier marqueur de gaine latérale (155) et un deuxième marqueur de gaine latérale (159).
